# EUROPEAN PATENT SPECIFICATION

(11) **EP 1 551 451 B1**
(45) Date of publication and mention of the grant of the patent: **25.02.2009**
(21) Application number: 03759205.2
(22) Date of filing: 27.08.2003
(51) Int. Cl.: A61K 39/02, A61K 39/00, A61K 39/108, A61K 39/295, A61K 45/00, A61K 39/116, A61K 39/12, C12N 15/09, C12P 21/04, C12N 15/82

(54) **USE OF ESCHERICHIA COLI HEAT LABILE TOXIN AS AN ADJUVANT IN POULTRY**
VERWENDUNG VON HITZELABILEM ESCHERICHIA-COLI-TOXIN ALS ADJUVANS BEI GEFLÜGEL
UTILISATION DE LA TOXINE THERMOLABILE D'ESCHERICHIA COLI EN TANT QU' ADJUVANT CHEZ LA VOLAILLE

(30) Priority: 27.08.2002 US 406359 P
(43) Date of publication of application: 13.07.2005
(73) Proprietor: Dow AgroSciences LLC, Indianapolis, Indiana 46268-1054 (US)
(72) Inventor: MILLER, Timothy, J., Lincoln, NE 68510 (US); FANTON, Matthew, J., Lincoln, NE 68516 (US)
(74) Representative: Fisher, Adrian John
(86) International application number: PCT/US2003/026721
(87) International publication number: WO 2004/020585

(56) References cited:
- WO-A1-96/06627
- US-A- 6 149 919
- US-B1- 6 395 964
- KHOURY C A ET AL: "A genetic hydrid of the Campylobacter jejuni flaA gene with LT-B of Escherichia coli and assessment of the efficacy of the hybrid protein as an oral chicken vaccine" AVIAN DISEASES, vol. 39, no. 4, 1995, pages 812-820, XP009054187 ISSN: 0005-2086
- RYAN E J ET AL: "Immunomodulators and delivery systems for vaccination by mucosal routes" TRENDS IN BIOTECHNOLOGY, ELSEVIER, AMSTERDAM,, GB, vol. 19, no. 8, 1 August 2001 (2001-08-01), pages 293-304, XP004254296 ISSN: 0167-7799
- GIULIANI ET AL: 'Mucosal adjuvanticity and immunogenicity of LTR72, a novel mutant of Escherichia coli heat-labile enterotoxin with partial knockout of ADP-ribosyltransferase activity' J. EXP. MED. vol. 187, no. 7, 06 April 1998, pages 1123 - 1132, XP002928610

## Description

The present invention generally relates to the field of immunology and provides adjuvant compositions and methods useful for vaccinating broiler chickens against mucosal pathogens. The invention particularly relates to the use of genetically modified plants that produce immunogenic peptides, protein, and adjuvants.

Oral vaccination has long been viewed as a convenient and non-traumatic method to administer vaccines. Edible vaccines are particularly attractive to the animal health industry because they would ideally combine the convenience of medication with established feeding practices in animals. Despite the success of oral polio vaccines since the 1960's, there has been very little commercialization of oral vaccines, especially vaccines that utilize non-viable antigens for stimulating the immune response. Transgenic plants provide a means to economically manufacture antigens for edible vaccines. The first reported work in this field is found in US Patent numbers 5,654,184; 5,679,380; and 5,686,079. Such transgenic plants provide the means to combine the features of utilizing accepted feeding practices of animals with the convenience of oral antigen delivery to mucosal tissue for vaccination.

For an edible vaccine to be successful, the complex environment of the digestive tract must be navigated. The mucosal associated lymphoid tissue (MALT) that is present in the digestive, respiratory and reproductive tracts of vertebrates is one of the most diverse, yet universal, immune systems in nature. Any antigen capable of stimulating an immune response in the digestive tract via gut associated lymphatic tissue (GALT) encounters a composite environment when entering the digestive tract. The oral cavity and other digestive organs contains a wide array of pH, enzymes, detergents, and commensal microorganisms that contribute in the digestion of food.

Commensal microorganisms are often more numerous than the number of cells making up the digestive tract itself. Savage, D. 1999. Mucosal Microbiota. Mucosal Immunity, eds (P. Orga, J. Mestecky, M. Lamm, W. Strober, J. Biennenstock, J. McGhee) Academic Press, Inc. pp 19-30. Despite this fact, these commensal microorganisms provide a useful function and are in a constant state of "tolerance" by the immune system of the GALT. Antigens face competition with food components and other environmental components that have been ingested and are at various stages of digestion. The immune system must continually survey the digestive, as well as the respiratory and reproductive tracts, for foreign versus self-antigens, including food antigens, which have features of "self" in that they are tolerized immunologically. Finally, an innate immune barrier composed of villiated and follicular epithelium lined with mucus having both passive and active transport mechanisms is in a constant flux two accommodate nutritional components and provide necessary secretions to the mucosal surface.

Despite the complex environment of the digestive tract, a very small amount of biologically active (gut-active) material can induce an immune response. Two of the most potent immune stimulators of the mucosal system, heat labile toxin (LT) from *Escherichia coli (E. coli)* and cholera toxin (CT) from *Vibrio cholera* (*V. cholera*)*,* can stimulate a serological response with just a few micrograms when given by oral gavage. Isaka, M., et al., 1998, Vaccine 16: 1620-1626; Rappuoli, R. et al., 1999; Immunol. Today. 20: 493-499; and Isaka, M., et al., 2000, Vaccine 18: 743-751. Thus, the mucosal surface can be breached successfully and with small amounts of biologically active protein such as LT and CT. However, due to their enteropathic toxigenic activity, LT and CT have not been proposed as oral adjuvants for safe use in humans or animals in their native forms.

One of the main features of mucosally active proteins like CT and LT, as opposed to a non-viable antigen, is that CT and LT are ligands for a gut receptor (GM1-ganglioside) that allows entry (invasivness) of these toxins in small concentrations. This is in contrast to a non-viable antigen that has no invasive quality and becomes diluted and subject to the digestive environment of the gut. Dogma supports that a non-invasive antigen requires high doses (milligram amounts) with multiple administrations to stimulate an immune response through the gut mucosal surface. However, the high antigen dose and multiple administrations are probably required to saturate the digestive environment. Since most of the antigen is degraded or absorbed, it may take a very small amount of antigen to sift through and stimulate an acquired immune response.

Because LT and CT are so pathogenic, they have been the object of substantial biochemical and genetic research aimed at reducing or eliminating their pathogenic properties while preserving adjuvant activity. LT and CT exhibit analogous tertiary protein structure in that both are composed of an oligomeric "B" subunit (LT-B and CT-B respectively) which has specificity for G1 ganglioside receptors and a single "A" subunit (LT-A and CT-A respectively) that has ADP ribosyl transferase activity. Research involving LT has produced numerous analogs that have been tested for their immonogenic and adjuvant properties in mammals. Several analogs of the LT toxin have been made by amino acid substitutions and deletions in the "A" subunit to attenuate the enteropathic activity without reducing the adjuvanting properties of the toxin (Ghiara et al., 1997, Infect. Immun., 65:4996-5002). There is, however, little information on the use of such toxins in birds either as adjuvants or immunogens.

Hoshi et al., 1995. Vaccine, 13: 245-252 and Meinersmann et al., 1993. Avian Dis., 37: 427-432, reported that oral administration of CT in avian species failed to increase systemic and mucosal humoral responses towards orally administered tetanus toxoid or inactivated infectious bursal disease virus. Takeda et al., 1996. Vet. Microbiol., 50: 17-25 demonstrated that intranasal and subcutaneous administration of CT-B was able to enhance the humoral response towards Newcastle Disease virus. Also, Vervelde et al., 1998. Vet. Imunol. Immunopath., 62: 261-272 reported that intra-intestinal, surgical application of CT in chickens increased the humoral response to intra-intestinal, surgical application *Eimeria* antigen. Therefore it was concluded that CT failed to produce adjuvating effects upon oral administration in birds even though surgical administration of CT to the intestine demonstrated binding to the intestinal epithelium.

Based on a large body of literature relating to the enteropathologic and adjuvant effects of LT and CT in a wide variety of mammals, it was anticipated that similar effects would be seen in birds. It was quite unexpected to discover that broiler chickens failed to display symptoms of diarrhea or other enteropathic effects while still producing an immunological response upon oral administration of native LT and CT toxin presented in a variety of formats ranging from purified toxin to *in situ*, plant-produced toxin.

Enteropathogenic *E. coli* expressing heat labile toxin has not been described for poultry, nor has the use of LT as a mucosal immunogen or adjuvant. Poultry have the ability to be immunized at a young age by virtue, in part, of a B-cell differentiation organ (Bursa of Fabricius) that is active before hatch. Dibner, J. J. et al., 1998. Applied Poultry Research 7: 427-436. Thus, birds immunized at day of age have a reduced load of feed, microorganisms and digestive enzymes in the gut to compete or degrade a non-viable antigen.

The studies described in this specification demonstrate that native LT and LT-B induce serological responses detected in serum IgG in as little as 21 days after inoculation of the toxin by either the intranasal (IN), subcutaneous, in ovo or oral routes. In a similar manner, transgenic tobacco NT-1 cells expressing native sequence LT-B and an attenuated LT-A analog induce serological responses via both the oral and IN routes without any adverse affects caused by the toxin or by the plant material. Furthermore, when administered by oral or Subcutaneous routes, native LT was unexpectedly found to be non-pathogenic for broiler chickens when given at doses higher than that reported to be lethal in mice and enteropathogenic in humans.

The invention can be summarized as a composition comprising an adjuvating amount of a protein selected from the group consisting of *E*. *coli* heat-labile toxin (LT) and *E*. *coli* heat-labile toxin analogs (LT analogs) for use as an adjuvant when vaccinating broiler chickens. The invention further consists of methods for vaccinating broiler chickens comprising administering an adjuvating amount of any of the claimed compositions to a broiler chicken especially when such compositions are produced by a transgenic plant and/or administered in the form of processed or unprocessed transgenic plant material.

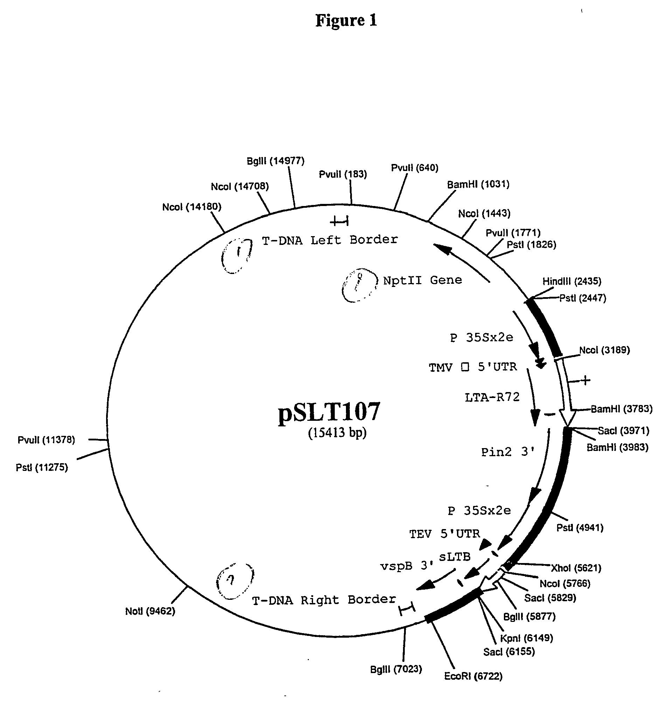

Figure 1. Map of pSLt107 used to transform NT-1 cells. The T-DNA left and right borders delimit the DNA to be transferred to plant cells, and flank expression cassettes for LT-A-R72 with transcription driven by the double-enhanced CaMV 35S promoter and terminated by the potato *pin2* 3' region; LT-B with transcription driven by the double-enhanced.CaMV 35S promoter and terminated by the soybean *vspB* 3' region; and *npt2* for selection of kanamycin resistant plants. Also note that pSLT 101, pSLT102, and SLT105 used to transform NT-1 cells described in the examples are identical to pSLT107 in all respects except for the LT-A coding region. The pSLT102 vector encoded the K63 LT-A mutant, the pSLT105 vector encoded the G192 LT-A mutant, and the pSLT107 vector encoded the R72 LT-A mutant described in Rappuoli, et. al., 1999. Immunol. Today. 20, 493-500. The pSLT101 vector encoded fully native LT biologically equivalent to *E*. *coli*-derived LT.

An immunogen is a non-self substance that elicits a humoral and/or cellular immune response in healthy animals such that the animal is protected against future exposure to the immunogen. Immunogens are typically pathogens such as viruses, bacterial and fungi that may be rendered nonpathogenic in some-fashion. Immunogens may also be antigenic portions of pathogens such as cell wall components, viral coat proteins, and secreted proteins such as toxins and enzymes, to name but a few. Immunogens also include recombinant cells such as plant cells and extracts of such cells that have been engineered to express and present immunoprotective antigens.

Vaccination and vaccinating is defined as a means for providing protection against a pathogen by inoculating a host with an immunogenic preparation of pathogenic agent, or a non-virulent form or part thereof, such that the host immune system is stimulated and prevents or attenuates subsequent host reactions to later exposures of the the pathogenic agent.

Administering or administer is defined as the introduction of a substance into the body of an animal and includes oral, nasal, ocular, rectal, *in ovo*, and parenteral (intraveneous, intramuscular, or subcutaneous) routes. More preferred routes of administration consistent with the present invention are oral and/or nasal administration, both of which can reach the gut mucosa, and *in ovo*. Oral administration is more highly preferred.

An effective or immunoprotective amount is defined as that quantity or mass of a substance that produces the desired consequence of protection against a disease challenge. Effective amounts will be apparent to those skilled in the in light of the data and information provided in this specification.

For purposes of this specification, an adjuvant is a substance that accentuates, increases, or enhances the immune response to an immunogen or antigen. Adjuvants typically enhance both the humor and cellular immune response but an increased response to either in the absence of the other qualifies to define an adjuvant. Moreover, adjuvants and their uses are well known to immunologists and are typically employed to enhance the immune response when doses of immunogen are limited or when the immunogen is poorly immunogenic or when the route of administration is sub-optimal. Thus the term 'adjuvating amount' is that quantity of adjuvant capable of enhancing the immune response to a given immunogen or antigen. The mass that equals an adjuvating amount will vary and is dependant on a variety of factors including but not limited to the characterisitcs of the immunogen, the quantity of immunogen administered, the host species, the route of administration, and the protocol for administring the immunogen. The adjuvating amount can readily be quantified by routine experimentation given a particular set of circumstances. This is well within the ordinarily skilled artisan's purview and typically employs the use of routine dose response determinations to varying amounts of administered immunogen and adjuvant. Responses are measured by determining serum antibody titers raised in response to the immunogen using enzyme linked immunosorbant assays, radio immune assays, hemagglutinations assays and the like.

In the case of LT a considerable body of working mammals has established that submicrogram per kilogram quantities not only elicit a strong immune response but also produce pathology. Most surprisingly, this was not shown to be the case in broiler chickens because up to 10 mg/kg of native LT administered orally failed to produce any signs of pathology yet still produced a substantial immune response in the form of high serum IgG titers. This is in contrast to studies showing that surgically implanted CT and antigen raised an immune response in the absence of pathology (Vervelde *et. al*., 1998. *Supra*) while CT-B was able to produce an immune response but no pathology (Takeda *et. al*., 1996*. Supra*)*.*

*E. coli* heat labile toxin (LT) has been well characterized by X-ray crystallography and consists of a multimeric protein. The holotoxin includes one 'A' subunit (LT-A) of molecular weight 27,000 Daltons which is cleaved into LT-A1 and LT-A2 by the proteases in the small bowel. The holotoxin also contains five 'B' subunits (LT-B), of molecular weight 11,600 Daltons each that link non-covalently into a very stable doughnut-like pentamer structure. Native LT from any source is preferred.

*E. coli* heat labile toxin analogs (LT analogs) are defined as any known native LT in which one or more amino acids have been substituted and reported in the literature. Several well-known LT analogs are those in which the residues naturally found at positions 63, 72, and 192 of the alpha sub-unit are substituted to lysine, arginine, and glycine respectively (K63, R72, and G192). Rappuoli, et. al., 1999. Immunol. Today. 20,493-500. Biologically active LT and LT analogs bind to GM1 ganglioside in an ELISA format, and are toxic to Y1 adrenal cells for *in vitro* cell-cytotoxicity tests.

Birds are susceptible to a wide variety of diseases for which the present inventions provides protective vaccination. The following is a list of some of the more commercially important avian diseases whose causative agents represent immunogens that are compatible with the present invention. This list in no way represents an exhaustive list of avian diseases applicable to the present invention. Newcastle disease, avain influenza, infectious bursal disease, coccidiosis, necrotic enteritis, airsacculitis, cellulitis, chicken anemia, larygnorhinotracheitis, infectious bronchitis, and Marek's disease. A preferred group of immunogens that provide protection against avian diseases and are consistent with the present invention is antigenic determinants of the Newcastle disease virus, the hemagglutinin neuraminidase protein of Newcastle disease virus, antigenic determinants of the avian influenza virus, the hemagglutinin protein of avian influenza virus.

Numerous methods are well known in the art for producing quantities of relatively pure polypeptides and proteins, including LT-A, LT-B, LT and LT analogs. Bacterial and yeast systems for producing recombinant polypeptides and proteins have been practiced for decades. More recently transgenic protein production systems have been developed that rely on insect, vertebrate, mammalian, or plant cells as the production vehicle. Production of an adjuvant such as LT, and optionally co-expression with an immunogen of choice, in a transgenic plant is particularly suited to the present invention because the transgenic plant material may be processed directly and fed to birds as a vaccine or administered intranasally

Transgenic plant is herein defined as a plant cell culture, plant cell line, plant, or progeny thereof derived from a transformed plant cell, tissue or protoplast, wherein the genome of the transformed plant contains exogenous foreign DNA, introduced by laboratory techniques, not originally present in a native, non-transgenic plant of the same strain. The terms "transgenic plant " and "transformed plant" have sometimes been used in the art as synonymous terms to define a plant whose DNA contains an exogenous DNA molecule. Also included in this definition are plants that have been tranformed transiently with heterologous DNA via viral vectors system that do not produce integrated transgenic events. This technology is well known in the art as represented in part by US Patent Numbers 5,846,795 and 5,500,360.

A preferred group of plants for use in the practice the present invention is plant cell cultures, potato, tomato, alfalfa, and duckweed. A more preferred group is plant cell cultures, and tomato, and plant cell cultures are most preferred. Preferred plant cell cultures include both monocot and dicot-derived cultures. Particularly preferred plant cell cultures are the tobacco cell cultures designated as NT-1 and BY-2 described by Toshiyuki et al., 1992. Int'l Rev. of Cytology, 132, 1-30.

There are many methods well know in the art for introducing transforming DNA segments into cells, but not all are suitable for delivering DNA to plant cells. Suitable methods are believed to include virtually any method by which DNA can be introduced into a cell, such as by *Agrobacterium* infection, direct delivery of DNA, for example, by PEG-mediated transformation of protoplasts (Omirulleh et al., Plant Molecular Biology, 21:415-428, 1993.), by desiccation/inhibition-mediated DNA uptake, by electroporation, by agitation with silicon carbide fibers, by acceleration of DNA coated particles, etc. In certain embodiments, acceleration methods are preferred and include, for example, microprojectile bombardment and the like.

Technology for introducingf DNA into cells is well-known to those of skill in the art. Four basic methods for delivering foreign DNA into plant cells have been described. Chemical methods (Graham and van der Eb, Virology, 54(02):536-539, 1973; Zatloukal, Wagner, Cotten, Phillips, Plank, Steinlein, Curiel, Birnstiel, Ann. N.Y. Acad. Sci., 660:136-153, 1992); Physical methods including microinjection (Capecchi, Cell, 22(2):479-488, 1980), electroporation (Wong and Neumann, Biochim. Biophys. Res. Conmmun. 107(2):584-587, 1982; Fromm, Taylor, Walbot, Proc. Natl. Acad. Sci. USA, 82(17):5824-5828,1985; U.S. Pat. No. 5,384,253) and the gene gun (Johnston and Tang, Methods Cell. Biol., 43(A):353-365, 1994; Fynan, Webster, Fuller, Haynes, Santoro, Robinson, Proc. Natl. Acad. Sci. USA 90(24):11478-11482, 1993); Viral methods (Clapp, Clin. Perinatol., 20(1):155-168, 1993; Lu, Xiao, Clapp, Li, Broxmeyer, J. Exp. Med. 178(6):2089-2096, 1993; Eglitis and Anderson, Biotechniques, 6(7):608-614, 1988; Eglitis, Kantoff, Kohn, Karson, Moen, Lothrop, Blaese, Anderson, Aνd. Exp. Med. Biol., 241:19-27, 1988); and Receptor-mediated methods (Curiel, Agarwal, Wagner, Cotten, Proc. Natl. Acad. Sci. USA, 88(19):8850-8854, 1991; Curiel, Wagner, Cotten, Birnstiel, Agarwal, Li, Loechel, Hu, Hum. Gen. Ther., 3(2):147-154, 1992; Wagner et al., Proc. Natl. Acad. Sci. USA, 89 (13):6099-6103, 1992).

The introduction of DNA into plant cells by means of electroporation is well-known to those of skill in the art. Plant cell wall-degrading enzymes, such as pectin-degrading enzymes, are used to render the recipient cells more susceptible to transformation by electroporation than untreated cells. To effect transformation by electroporation one may employ either friable tissues such as a suspension culture of cells, or embryogenic callus, or immature embryos or other organized tissues directly. It is generally necessary to partially degrade the cell walls of the target plant material to pectin-degrading enzymes or mechanically wounding in a controlled manner. Such treated plant material is ready to receive foreign DNA by electroporation.

Another method for delivering foreign transforming DNA to plant cells is by microprojectile bombardment. In this method, microparticles are coated with foreign DNA and delivered into cells by a propelling force. Such micro particles are typically made of tungsten, gold, platinum, and similar metals. An advantage of microprojectile bombardment is that neither the isolation of protoplasts (Cristou et al., 1988, Plant Physiol., 87:671-674,) nor the susceptibility to *Agrobacterium* infection is required. An illustrative embodiment of a method for delivering DNA into maize cells by acceleration is a Biolistics Particle Delivery System, which can be used to propel particles coated with DNA or cells through a screen onto a filter surface covered with corn cells cultured in suspension. The screen disperses the particles so that they are not delivered to the recipient cells in large aggregates. For the bombardment, cells in suspension are preferably concentrated on filters or solid culture medium. Alternatively, immature embryos or other target cells may be arranged on solid culture medium. The cells to be bombarded are positioned at an appropriate distance below the macroprojectile stopping plate. In bombardment transformation, one may optimize the prebombardment culturing conditions and the bombardment parameters to yield the maximum numbers of stable transformants. Both the physical and biological parameters for bombardment are important in this technology. Physical factors are those that involve manipulating the DNA/microprojectile precipitate or those that affect the flight and velocity of either the microprojectiles. Biological factors include all steps involved in manipulation of cells before and immediately after bombardment, the osmotic adjustment of target cells to help alleviate the trauma associated with bombardment, and also the nature of the transforming DNA, such as linearized DNA or intact supercoiled plasmids.

*Agrobacterium-*mediated transfer is a widely applicable system for introducing foreign DNA into plant cells because the DNA can be introduced into whole plant tissues, eliminating the need to regenerat an intact plant from a protoplast. The use of *Agrobacterium-*mediated plant integrating vectors to introduce DNA into plant cells is well known in the art. See, for example, the methods described in Fraley et al., 1985, Biotechnology, 3:629; Rogers et al., 1987, Meth. in Enzymol., 153:253-277. Further, the integration of the Ti-DNA is a relatively precise process resulting in few rearrangements. The region of DNA to be transferred is defined by the border sequences, and intervening DNA is usually inserted into the plant genome as described in Spiehmann et al., 1986, Mol. Gen. Genet., 205:34; Jorgensen et al., 1987, Mol. Gen. Genet., 207:471.

Modem *Agrobacterium* transformation vectors are capable of replication in *E*. *coli* as well as *Agrobacterium,* allowing for convenient manipulations as described (Klee et al., 1985). Moreover, recent technological advances in vectors for *Agrobacterium*-mediated gene transfer have improved the arrangement of genes and restriction sites in the vectors to facilitate construction of vectors capable of expressing various proteins or polypeptides. The vectors described (Rogers et al., 1987), have convenient multi-linker regions flanked by a promoter and a polyadenylation site for direct expression of inserted polypeptide coding genes and are suitable for present purposes. In addition, *Agrobacterium* containing both armed and disarmed Ti genes can be used for the transformations.

Transformation of plant protoplasts can be achieved using methods based on calcium phosphate precipitation, polyethylene glycol treatment, electroporation, and combinations of these treatments (see, e.g., Potrykus et al., 1985. Mol. Gen. Genet., 199:183 and Marcotte et al., 1988. Nature, 335:454). Application of these systems to different plant species depends on the ability to regenerate the particular species from protoplasts.

### Example 1

### Materials

The plant-optimized sequence encoding the LT-B gene of *E. coli* strain H10407 is know in the art (Mason et al., 1998. Vaccine 16:1336-1343). The plant-optimized sequence encoding the LT-A gene of *E. coli* strain H10407 was described in WO/2000/37609 which was originally filed as US Provisional Application Number 60/113,507. WO/2000/37609 describes the construction of three binary T-DNA vectors (pSLT102, pSLT105, pSLT107) that were used for *Agrobacterium tumefaciens-*mediated plant cell transformation of *Nicotiana tabacum* NT-1 cells in Example 2. The resulting transformed NT-1 cell lines (SLT102, SLT105 and SLT107) expressed and accumulated fully assembled LT and LT analogs comprised of LT-B and modified forms of the LT-A subunit. Figure 1 illustrates pSLT107, which contains a modified LT-A gene that replaces Ala72 with Arg72. SLT102 and SLT105 expression products were identical except that they contained different alterations in the LT-A gene (Ser63 to Lys63 in pSLT102; Arg192 to Gly192 in pSLT105. These lines contain an undetermined number of copies of the T-DNA region of the plasmids stably integrated into the nuclear chromosomal DNA. The transgenic NT1 cells accumulated LT-B subunits that assembled into ganglioside-binding pentamers, at levels up to 0.4% of total soluble protein as determined by ganglioside-dependent ELISA. The transgenic NT1 cells also accumulated modified LT-A subunits, some of which assembled with LT-B pentamers as determined by ganglioside-dependent ELISA using LT-A specific antibodies.

Also, plasmid pQETHK, pJC217 and pCS96 were obtained. pQETHK is a plant optimized coding sequence of LTB cloned into Quiagens pQE60® vector; pJC217 (Cardenas, et al., 1993, Inf. and Imm. 61:4629-4636) that contains the *E. coli* derived sequence of LTB and the non-coding region of LTA cloned into pUC8. pCS96 expresses both the LTB and LTA subunit genes of *E. coli.* Each plasmid in DH5α or JM83 host strain of *E. coli* was grown in LB media (Gibco/BRL) using 50µg/ml of ampicillin for selection

Native LT was isolated by growing 1-2 liters of pCS96 in DH5α overnight in LB media containing 50ug/ml ampicillin. The cells were pelleted using a Heraeus Megafuge (20 minutes at 4000rpm), resuspended in 200 ml of TE buffer (50mM Tris pH 7.2, 1 mM EDTA), centrifuged 20 minutes at 4000 rpm and resuspended in 100 ml of TE Buffer and stored at - 80°C. The resuspended pellet was disrupted using a Branson 450 sonifier with a flat replaceable tip at output control of 8, duty cycle 60, for 10 minutes on ice. Preparations were then centrifuged at 10,000 rpm for 30 minutes at 2-7 °C using a J2-21 Beckman centrifuge and JA-20 Beckman rotor. The supernatant was transferred to new tubes and centrifuged at 20,000 RPM for 1 hour. The 20,000 RPM supernatant was transferred to dialysis tubing (6000-8000 MWCO) and dialyzed against 2L TEN for 4 days (TEN was changed daily). After dialysis, the sample was passed overe a TEN equilibrated immobilized galactose affinity column at a flow rate of ∼10 ml/hour. The column was washed with several column volumes of TEN buffer and the bound LT was eluted with 1M D (+) galactose. Fractions containing the LT were verified by polyacrylamide gel electrophoresis, pooled and stored at -80 °C.

### Example 2

### Preparation of Transgenic Nicotiana tabacum Expressing LT

Three to 4 days prior to transformation, a 1 week old NT-1 culture was sub-cultured to fresh medium by adding 2 ml of the NT-1 culture into 40 ml NT-1 media. The sub-cultured was maintained in the dark at 25 ± 1°C on a shaker at 100 rpm.

### NT-1 Medium

| *Reagent* | *Per liter* |
|---|---|
| MS salts | 4.3 g |
| MES stock (20X) | 50 ml |
| B1 inositol stock (100X) | 10 ml |
| Miller's 1 stock | 3 ml |
| 2,4-D (1 mg/ml) | 2.21 ml |
| Sucrose | 30 g |
| pH to 5.7 ± 0.03 | |

| | |
|---|---|
| B1 Inositol Stock (100x)(1 liter) Thiamine HCl (Vit B1) - 0.1 g MES (20x) (1 liter) MES (2-N-morpholinoethanesulfonic acid) -10 g Myoinositol - 10 g Miller's I (1 liter) KH₂PO₄ - 60 g | |

*Agrobacterium tumefaciens* containing the expression vector of interest was streaked from a glycerol stock onto a plate of LB medium containing 50 mg/l spectinomycin. The bacterial culture was incubated in the dark at 30°C for 24 to 48 hours. One well-formed colony was selected, and transferred to 3 ml of YM medium containing 50 mg/L spectinomycin. The liquid culture was incubated in the dark at 30°C in an incubator shaker at 250 rpm until the OD₆₀₀ was 0.5 - 0.6. This took approximately 24 hrs.

| **LB Medium** | | **YM Medium** | |
|---|---|---|---|
| Reagent | Per Liter | Reagent | Per Liter |
| Bacto-typtone | 10 g | Yeast extract | 400 mg |
| Yeast extract | 5 g | Mannitol | 10 g |
| NaCl | 10 g | NaCl | 100 mg |
| Difco Bacto Agar | 15 g | MgSO₄•7H₂0 | 200 mg |
| | | KH₂PO₄ | 500 mg |

(Alternatively, YM in powder form can be purchased (Gibco BRL; catalog #10090-011). To make liquid culture medium, add 11.1 g to 1 liter water.)

On the day of transformation, 1 µl of 20 mM acetosyringone was added per ml of NT-1 culture. The acetosyringone stock was made in ethanol the day of the transformation. The NT-1 cells were wounded to increase the transformation efficiency. For wounding, the suspension culture was drawn up and down repeatedly (20 times) through a 5 ml wide-bore sterile pipet. Four milliliters of the suspension was transferred into each of 10, 60 x 15 mm Petri plates. One plate was set aside to be used as a non-transformed control. Approximately, 50 to 100 µl of *Agrobacterium* suspension was added to each of the remaining 9 plates. The plates were wrapped with parafilm then incubated in the dark on a shaker at 100 rpm at 25 ± 1°C for 3 days.

Cells were transferred to a sterile, 50 ml conical centrifuge tube, and brought up to a final volume of 45 ml with NTC medium (NT-1 medium containing 500 mg/L carbenicillin, added after autoclaving). They were mixed, then centrifuged at 1000 rpm for 10 min in a centrifuge equipped with a swinging bucket rotor. The supernatant was removed, and the resultant pellet was resuspended in 45 ml of NTC. The wash was repeated. The suspension was centrifuged, the supernatant was discarded, and the pellet was resuspended in 40 ml NTC. Aliquots of 5 ml were plated onto each Petri plate (150 x 15 mm) containing NTCB10 medium (NTC medium solidified with 8g/l Agar/Agar; supplemented with 10 mg/l bialaphos, added after autoclaving). Plates were wrapped with parafilm then maintained in the dark at 25 ± 1C. Before transferring to the culture room, plates were left open in the laminar flow hood to allow excess liquid to evaporate. After 6 to 8 weeks, putative transformants appeared. They were selected and transferred to fresh NTCB5 (NTC medium solidified with 8g/l Agar/Agar; supplemented with 5 mg/l bialaphos, added after autoclaving). The plates were wrapped with parafilm and cultured in the dark at 25 ± 1°C.

Putative transformants appeared as small clusters of callus on a background of dead, non-transformed cells. These calli were transferred to NTCB5 medium and allowed to grow for several weeks. Portions of each putative transformant were selected for ELISA analysis. After at least 2 runs through ELISA, lines with the highest antigen levels were selected. The amount of callus material for each of the elite lines was then multiplied in plate cultures and occasionally in liquid cultures.

The resulting transformed NT-1 cell lines SLT102, SLT105 and SLT107 expressed and accumulated the *E. coli* heat-labile enterotoxin B subunit (LT-B) and modified forms of the LT-A subunit. The expression products from SLT102, 105 and 107 were identical except that they contain different alterations in the LT-A gene. Another NT-1 line, SLT101, expressed the native LT-A and LT-B genes and was equivalent in all respects to *E. coli*-derived LT. These lines contain an undetermined number of copies of the T-DNA region of the plasmids stably integrated into the nuclear chromosomal DNA. All transgenic NT-1 cells accumulated LT-B subunits that assembled into ganglioside-binding pentamers, at levels up to 0.4% of total soluble protein as determined by ganglioside-dependent ELISA. All transgenic NT-1 cells also accumulated native (SLT101) or modified LT-A (SLT 102, SLT105 and SLT107) sub-units that assembled with LT-B pentamers as determined by ganglioside-dependent ELISA using LT-A specific antibodies.

### Example 3

### Preparation of Inoculum

Buffers and Reagents: Ampicillin was obtained from Sigma (Lot No.14H0041), and a 100 mg/ml stock was made in sterile water. Tryptone was obtained from Fisher Biotech (Lot No. 109756JE). Yeast extract was obtained from Difco (Lot No. 132384JD). Sodium chloride (Lot No.49H0265), D(+) galactose (Lot No. 46HO3561) MES (2-[morpholino]ethanesulfonic acid) (Lot No. 29H54281), and sodium hydroxide (Lot No. 30K0229) were obtained from Sigma. Coomassie Brilliant Blue G-250 was obtained from Bio-Rad. Methanol was obtained from VWR (Lot No. 38274842, glacial acetic acid from EM Sciences Lot No. K27260700, and ethyl alcohol Lot No. DUO9723BU from Aldrich Chemical. Phosphate buffered saline (PBS) was prepared as 0.14M sodium chloride, 1.5 mM potassium phosphate monobasic, 2.5 mM potassium chloride, and 6 mM sodium phosphate dibasic, pH 7.2 (BRL/Gibco).

Transformed NT-1 cells were maintained as callus on agar plates prepared from NT-1 media containing 0.8% agar. The components of the media include 2.5 mM MES, 1.2 mM dibasic potassium phosphate (Lot No. 47HO811), 0.1% (w/v) myoinositol (Lot No.49H039025), 0.001 % (w/v) thiamine HCl (Lot No.107H02785), 0.4% (w/v) MS salts (Lot No.470803), 3% (w/v) sucrose (Lot No.47H0803), and 0.8% (w/v) agar-agar (L#390906), all from Sigma, and 0.22% (v/v) 2,4-D (Lot No.107091) from Gibco. Suspension cells as well as agar plates also contained 50ug/ml of kanamycin (L#129HO8941, Sigma) as required to evaluate the selection for the NT-1 transformed recombinant DNA. Callus was passed by taking a sterile pipette to break the callus and transferring a small amount of the callus (0.5 cm³) to a fresh plate. To produce suspension cultures of the NT-1 cells, the callus was broken with a pipette and several pieces of the callus were transferred to NT-1 media in an Erlenmeyer flask, without the agar, and placed in a gyratory incubator at 28-30°C. Cells were harvested by several methods 6-12 days after passage. Whole wet cells were obtained by holding the shaker flask stationary to allow cells to settle, followed by decanting the media. Sonicated whole wet cells were obtained as described above by resuspending the wet cells in extraction buffer containing 50 mM sodium ascorbate, 1mM EDTA, 1mM PMSF, and 0.1% Triton X-100 (all from Sigma), prepared in phosphate buffered saline pH 7.2. The cells were then broken open using a Branson 450 sonifier with a flat replaceable tip at output control of 8, duty cycle 60 for 10 minutes on ice. Supernatant and pellet from sonicated wet cells were prepared by centrifuging the sonicated preparation 20-30 min at 3400 rpm using a Beckman GPR centrifuge. Whole dried cells were prepared by filtering whole wet cells using a Buchner funnel lined with Spectramesh; the packed cells were spread onto a Spectramesh sheet in a shallow plastic tray, then placed in a food dehydrator overnight.

For on-feed treatments, the whole cells, dried cells or sonicated wet cells previously quantified for target antigen content were placed directly into feeding bowls. However, consumption of the target antigen was more efficient by broiler chicks if the whole wet cells, dried cells or sonicated cells were first mixed with feed (6 grams for broiler chicks 1 day of age) and placed in a single feeding bowl per cage of "X" birds. For intranasal (IN) inoculation the wet cells, dried cells or sonicated cells were suspended in PBS until a consistency was obtained to allow pipetting of 25 ul into the bird's nostril. Typically a cell mass to volume ratio needed for IN inoculation was 1 part cell to 2 parts extraction buffer.

### Example 4

### Vaccine Administration and Treatments

Broiler chicks were obtained from Stover Hatchery, Stover MO. These chicks are byproduct males or mixed sex chicks hatched for seeding small broiler operations. Chicks arrived by express mail overnight and were immediately placed into brooder Petersime cages (7 per cage). The number of chicks per treatment was based on a completely randomized design using repeat measurements. Any excess chicks were placed randomly in individual cages and were utilized to replace chicks that died from shipping or placement stress. Water was added *ad libitum* immediately upon seeding birds in cages. To fast birds, feed was withheld overnight and inoculation made the following morning, or feed was added *ad libitum* overnight and then withheld in the morning for 5 hours and inoculation made in the afternoon. For on feed inoculations, the plant derived antigen was slurried with a minimum amount water and then mixed with feed to make a clumpy paste. The feed was added to a bowl and the bowl was placed in the cage to allow access for all birds. For gavage treatment, a one-inch gavage needle fixed to a syringe was used to inoculate directly into the esophagus or crop. The intranasal route of administration was done by inoculating 25 ul directly into the nostrils. Chicks were generally between 18 and 30 hours old at inoculation, thus all clinical trials start with chicks at 1 day of age on Trial Day 0.

### Example 5

### Quantitative ELISA

Nunc Maxisorp 96-well microtiter ELISA plates were coated with 5 ug/well of mixed GM1 ganglioside in 0.01 M borate buffer using 100 ul per well; plates were incubated at room temperature overnight. The plates were washed 3 times with PBS-Tween® (1X containing 0.05% Tween 20, Sigma Lot No.120K0248). Each well was then incubated one hour at 37°C with 200 ul of blocking buffer containing 5% (w/v) non-fat dried milk in PBS- 0.05% Tween 20. The wells were washed 1X with 250 ul/well using PBS-Tween 20. Reference antigen and sample antigens were mixed with blocking buffer before adding to plates. LT reference antigen and LT-B reference antigen were diluted to 50ng/ml in the first well while samples were prediluted at several different starting dilutions. Samples were added to the plate by applying 200ul of sample in row A and 100 ul of blocking buffer to remainder rows. Mixing and transferring 100ul per well made serial 2-fold dilutions. Plates were then incubated 1h at 37°C, washed 3X in PBS-Tween and 100ul of diluted antisera in blocking buffer was added per well and incubated 1h at 37°C. The plates were washed 3X in PBS-Tween and then 100ul of antibody conjugate was added and incubated 1h at 37°C. The plates were washed 3X in PBS-Tween and 50ul of TMB substrate was added to each plate and TMB stop solution was added at 20 minutes post addition of substrate. Optical density at 450 nm wavelength was determined using a Tecan Sunrise Plate reader. Data were transported and displayed using Tecan Magellan Software. Linear regression and quantitation analyses were done using Microsoft Excel 2000 verson 9.0.3821 SR-1.

### Example 6

### Serum ELISA

Blood was collected by decapitation (birds 0-7 days of age) or by venipuncture in the wing web or jugular vein. Birds were euthanized by cervical dislocation or by CO₂ exposure for 1-5 minutes prior to decapitation. The blood was transported from the animal facility to the laboratory and placed at 2-7 °C for 45 minutes to advance and condense the blood clot. The blood samples were transferred to a 37°C water bath for 10 minutes and then centrifuged for 20 minutes at 2500 rpm using a Beckman GPR centrifuge at 2-7°C. The serum was aseptically removed from each tube, 0.5-1.5ml was aliquoted to a cryotube (Nunc) and stored at -18°C until used. For serum ELISA, the ganglioside adsorption step utilized 1.5ug/ml with incubation overnight at

2-7 °C. The plates were washed 3X with PBS-Tween and blocked with 200 ul/well of 3% nonfat dried milk in PBS-Tween. To titer antibody per serum sample, after the gangliside is adsorbed, 100ul of LT-B or LT at 2.5ug/ml in blocking buffer is added per well and incubated 1h at 37°C. The plates were washed 3X with PBS-Tween and then 200 ul of the serum sample diluted in blocking buffer was added to Row A and 100 ul of blocking buffer was added to the remaining rows. Starting dilution for serum was 1:10 in blocking buffer unless specified otherwise. After two-fold serially dilutions of the serum samples, the plates were incubated 1h at 37°C and then washed 3X in PBS-Tween. Goat anti chicken antibody, pretitered for optimal binding, and chromagen were added and incubated 1h at 37°C. Plates were washed and 100u1 of ABTS was added and incubated until the initial dilution of the positive control provided a 0.7 to 1.0 absorbance at 405/492 dual wavelength using a Tecan Sunrise^{™} plate reader.

Data was transported and displayed using Tecan Magellan Software. Linear regression and quantitation analyses were done using Microsoft Excel 2000 version 9.0.3821 SR-1. The serum geometric mean titer (GMT) was determined for each treatment group using Microsoft Excel 2000 version 9.0.3821 SR-1. Background ELISA titers of < 10 were given a value of 1 for these calculations. Difference in least squares means for treated birds from controls was determined using least squares analysis. A treatment was passed as effective if there was a significant difference of a treatment group with the non-vaccinated non-challenged control group.

### Example 7

### Y1 Adrenal Cell Assay

Y1 adrenal cells from mice were purchased from ATCC (CCL-79, L#1353400). The cell vial was thawed at 37°C and placed into a 25 cm² T-flask (Corning) containing 10 ml of growth media consisting of 15% donor horse serum (Quad-5 L# 2212), 2.5% fetal bovine serum (JRH L# 7N2326), 1% glutamax-1 (Gibco L# 1080323) in F-12K media (Gibco L# 1089716). Cells were incubated at 37°C in 5% CO₂. Cells were maintained in this growth media at each passage and for LT and CT cytotoxicity assays. To assay, the cells are passed onto 96 well cell culture plates (Nunc) and allowed to reach 80% confluence. LT or CT toxin is diluted to 1ug/ml in F-12K growth media. The toxin is further diluted by two fold serial dilutions on a 96 well microtiter plate by adding 100ul of the prediluted sample to row A of the plate. Two fold serial dilutions are then made by transferring 50 ul of the sample in row A to 50 ul of growth media in the next well. Each dilution of the sample is transferred to 1-4 wells of Y1 adrenal cells depending on availability of samples or cells. The end point titer of CT or LT toxin is the ug/ml required to obtain 50% cytotoxicity (cell death). Guidry, J. J., Cardenas, L., Cheng, E. and J. D. Clements. 1993. Role of receptor binding in toxicity , immunogenicity and adjuvanticity of Escherichia coli heat-labile enterotoxin. Inf. and Imm. 65: 4943-4950.

### Example 8

### Challenge of Broiler Birds with Native LT

Broiler chicks were housed 5-6 birds per cage until 10 or 21 days of age. In a pilot study, 16 ten-day-old chicks were divided into three groups (5 birds uninoculated, 5 birds treated with 100ug/bird, 5 birds treated with 200 ug/bird) and inoculated subcutaneously with *E. coli* native LT. The LT toxin had previously been determined to be in active form by titration on mouse Y1-adrenal cells as described above. Birds were observed every hour for 8 hours post inoculation for clinical signs. At 1, 2, 4, 8, 24 and 26 hours post inoculation, birds were sacrificed and a gross necropsy was conducted. Weights and lesions of critical organs, including intestine, liver, kidney, spleen and adrenal, as well as body weights were recorded.

For the second study, 84 twenty-one day old birds were divided into 6 groups of 16 birds each and inoculated subcutaneously with 0,10, 50, 100, 200 or 400 ug of native LT holotoxin per bird. At 10, 24 and 48 hours post inoculation, 5 birds from each group were scored for body weights, gross pathology, fecal consistency and overall health. All birds were provided feed and water *ad libitum* throughout the study.

### Example 9

### Serological Response to Oral Administered CT-B in Broiler Chicks

Cholera toxin subunit B (CT-B) was added to feed or inoculated directly into chickens using various formats. For this study, three inoculations were made at 1, 14 and 28 days of age; blood was collected for antibody analysis at day 1, 14, 28 and 35 days of age. The data revealed several observations previously described for mammals, although not described for chickens. A measurable serological response was easily detected at 28 days of age or two weeks after the second vaccination at 14 days of age. Thus, only two doses were required to induce a detectable response. However, providing a third dose at 28 days of age enhanced the response. Both intranasal (IN) and on-feed (OF) inoculations induced serological responses; the highest titer (5407) occurred with an inoculum of 20 ug of CT-B delivered as an aqueous 25 ul dose to each nostril of the beak. Oral delivery for CT-B also provided a good response whether delivered as a 20 ug dose by gavage (titer 1790) or by 40 ug sprayed directly (top dressing) onto feed (titer 365). As observed for mammals, the IN route using CT-B provides a good serological response. (Verweij, et. al., 1998. Vaccine 16:2069-2076 and Isaka, et al. 1998. Vaccine 16: 1620-1626.) However, the dose volume needs to be small to allow entry through the nostril. In this case, purified CT-B toxin could be produced as a stock of 2mg/ml, which allowed dilution to the appropriate dose of 20 ug to be delivered in 25 ul. The spray application using 20 ug in 6 grams of feed, or about 0.3 ppm, provided a good titer. Considering the dilution of the CT-B on feed and the response that ensued, dosing by oral route appears to provide a good stimulation of the GALT whether inoculated directly into the esophagus or through *ad libitum* consumption of 6-gram inoculums. As a comparison, a 2 ug dose of CT-B given IN or 40 ug given orally on feed in chickens is very similar to the dose that will stimulate a mucosal response in mice. The dose shown effective for CT-B in this study is also comparable to the amount of antigen used in conventional vaccines for animals delivered by intramuscular (IM) or subcutaneous (SC) methods. In addition, no adverse side effects from CT-B were observed for these birds; the weight gain and feed consumption were normal which supports the safe use of transgenic derived plant antigens as oral vaccines.

### Example 10

### Serological Response to LT from Tobacco in Chickens

Utilizing the dose range determined effective for CT-B, a second study was performed to evaluate whether native *E. coli* heat labile toxin (LT) or LT-B, as well as a gene modified heat labile toxin could provide a similar response. In this study 40 ug of toxin as measured by the LT-B quantitative ELISA was administered in three doses in a similar time frame as the CT-B study described above. Table 1 provides the results of tobacco-derived LT (SLT) and native toxin administered by on feed method or oral vaccination. All treatment groups responded with the exception of SLT supernatant. "SLT whole" samples (titer 844) and "SLT sonicate" (titer 557) provided the highest responses in this study.

**Table 1**

| Plant-derived LT Compared to Bacterial-derived LT, LT-B and CT-B | | | | |
|---|---|---|---|---|
| **Treatment Group** | **Day 0 GMT** | **Day 14 GMT** | **Day 28 GMT** | **Day 34 GMT** |
| PBS | 10^{a} | <10^{a} | <10 | <10 |
| NT sonicate | <10^{a} | <10^{a} | <10 | <10 |
| NT dried - mixed | <10 | <10 | <10 | <10 |
| CT-B - sprayed | <10 | <10 | 77 | 266 |
| LT-B - sprayed | <10 | <10 | 30 | 171 |
| LT- sprayed | <10 | <10 | 32/53 | 133/226 |
| SLT whole - mixed | <10 | <10 | 260 | 844 |
| SLT sonicate - mixed | <10 | <10 | 130 | 577 |
| SLT pellet - mixed | <10 | <10 | 30 | 272 |
| SLT sup - mixed | 80 | <10 | <10 | <10 |
| SLT dried - mixed | <10 | <10 | 20 | 96 |
| SLT sonicate - mixed O/N | <10 | <10 | 12 | 72 |

The "SLT whole" were whole wet cells isolated by simply allowing NT-1 production flasks to settle, decanting the media and using the remaining whole wet cells and residual media to be mixed with feed. "SLT sonicate" was whole wet cells that were sonicated first to disrupt the cell wall and then mixed with feed. Both of the tobacco derived SLT samples induced a better serological response than native LT or CT-B when applied directly to feed. These results support tobacco cell-derived LT (SLT) and native LT as good mucosal antigens in chickens. Futhermore, Table 2 shows that tobacco derived SLT provided no harmful side effects as measured by weight gain which provides data in support of safe vaccination of chickens by the oral route using plant or native LT. In addition, native LT toxin provided at 40 ug at 1, 14 and 28 days of age provided no visible enteropathogenic effects (diarrhea, discomfort, dehydration) of treated birds at any age. The mass of LT per kg of body weight is estimated to be 0.6mg of LT/kg of body weight, which is well above lethal doses observed for mice (Gill, M. D. 1982. Bacterial toxins; a table of lethal amounts. Microbiol. Reviews. 46: 86-94.) and support the concept that native LT can be used safely in chickens .

**Table 2**

| Average total body weight per bird per treatment | | | | |
|---|---|---|---|---|
| Treatment | Average weight Day 0 (Kg) | Average weight Day 14 (Kg) | Average weight Day 28 (Kg) | Average weight Day 34 (Kg) |
| PBS control | 0.038 | 0.29 | 1.06 | 1.34 |
| NT sonicate-mixed | 0.040 | 0.30 | 1.06 | 1.39 |
| NT dried-mixed | 0.039 | 0.32 | 1.04 | 1.36 |
| CT-B-sprayed | 0.040 | 0.30 | 1.04 | 1.36 |
| LT-B-sprayed | 0.038 | 0.29 | 0.98 | 1.3 |
| LT-sprayed | 0.040 | 0.30 | 1.02 | 1.32 |
| SLT whole-mixed | 0.040 | 0.29 | 0.94 | 1.28 |
| SLT sonicate-mixed | 0.040 | 0.30 | 1.00 | 1.3 |
| SLT pellet-mixed | 0.038 | 0.30 | 1.04 | 1.38 |
| SLTsupertate-mixed | 0.040 | 0.33 | 0.92 | 1.32 |
| SLT dried-mixed | 0.040 | 0.32 | 1.08 | 1.42 |
| SLT sonicate-mixed and dried overnight | 0.038 | 0.33 | 1.12 | 1.41 |

### Example 11

### Onset to Serological Response and Duration of Serological Response to SLT

The previous two studies demonstrated that native *E. coli* or tobacco-derived heat labile toxin induces a strong serological response in broiler chicks. A study was conducted to examine the earliest day of age that a response could be measured in broiler chicks and the duration of the response. In this study a combination of dose and age of bird was used to examine response to SLT. Table 3 shows the treatment groups, age of bird at dosing and amount of antigen used at each dose for this study. The second dose was lower due to a low yield of antigen, which translated to a lower antigen dose when distributed among groups. The IN dose was approximately 100 ng; the low dose is due to the fact that the SLT antigen is diluted by the tobacco cell mass and the fact that resuspension of SLT cells must be made homogenous enough to allow application to the nostril of a one day old bird. A suspension of NT-1 cells diluted enough to allow transfer through a pipette provided a very small inoculum for the first dose. Despite this fact, when boosted by the second dose on feed, these birds responded, indicating that a small dose is adequate to prime birds to LT antigen by the IN route.

**Table 3**

| Treatment | 1° Dose (ug) | 2° Dose (ug) | 3° Dose (ug) |
|---|---|---|---|
| 1. In/Oral NT Control Cells (Days 0, 14, 28) | 0 | 0 | 0 |
| 2. On Feed NT Control Cells (Days 0, 14, 28) | 0 | 0 | 0 |
| 3. On Feed SLT102 Cells (Days 0, 14, 28) | 5 | 3 | 3 |
| 4. On Feed SLT102 Cells (Days 0, 14) | 5 | 3 | N/A |
| 5. On Feed SLT102 Cells (Days 0, 7) | 5 | 3 | N/A |
| 6. On Feed SLT102 Cells (Days 0, 7, 14) | 5 | 3 | 3 |
| 7. On Feed SLT102 Cells (Days 0, 14, 28) | 40 | 25 | 25 |
| 8. On Feed SLT102 Cells (Days 0, 14) | 40 | 25 | N/A |
| 9. On Feed SLT102 Cells (Days 0, 7) | 40 | 25 | N/A |
| 10. On Feed SLT102 Cells (Days 0, 7, 14) | 40 | 25 | 25 |
| 11. In/Oral SLT102 Cells (Days 0, 14, 28) | 100 (ng) | 25 | 25 |
| 12. In/Oral SLT102 Cells (Days 0, 7, 14) | 100 (ng) | 25 | 25 |

Table 3A shows the serological responses. The earliest age for detecting a serological response was 21 days regardless of whether the dose was provided at 0, 7 or 14 days of age. In groups 3, 7 and 11 a third dose was provided at 28 days of age but was not needed to provide a detectable titer. The response detected by 21 days of age lasted through 42 days of age, consistent with the market age (35-65 days) of broiler birds.

The best serological response was observed when only two doses were provided to the bird and in both cases the first inoculation was at 1 day of age and the second inoculation was at 7 days of age. Because chicks are typically inoculated *in ovo* at three days before hatch or at one day of age in the hatchery, the ability to prime the birds at one day of age can be adapted to vaccination practices for the broiler industry.

**Table 3A**

| Onset to Serological Response and Duration of Response | | | | | | | | |
|---|---|---|---|---|---|---|---|---|
| Treatment | Day 0 GMT | Day 7 GMT | Day 14 GMT | Day 18 GMT | Day 21 GMT | Day 28 GMT | Day 35 GMT | Day 42 GMT |
| 1 | 14 | <10 | <10 | <10 | <10 | <10 | <10 | <10 |
| 2 | 40 | <10 | <10 | <10 | <10 | <10 | <10 | <10 |
| 3 | 20 | <10 | <10 | <10 | <10 | <10 | <10 | <10 |
| 4 | 28 | <10 | <10 | <10 | <10 | 17 | <10 | <10 |
| 5 | 20 | <10 | <10 | <10 | <10 | <10 | <10 | <10 |
| 6 | 20 | <10 | <10 | <10 | <10 | 13 | <10 | <10 |
| 7 | 20 | <10 | <10 | <10 | <10 | 22 | 65 | 49 |
| 8 | 20 | <10 | <10 | <10 | 26 | 61 | 78 | 68 |
| 9 | 28 | <10 | <10 | 15 | 36 | 116 | 335 | 453 |
| 10 | 28 | <10 | <10 | <10 | 26 | 160 | 394 | 292 |
| 11 | 14 | <10 | <10 | <10 | <10 | <10 | 16 | 21 |
| 12 | 20 | <10 | <10 | <10 | 19 | 90 | 143 | 108 |

### Example 12

### Treatment of Broiler Birds with Native LT from E. coli

Tables 4 and 5 provide the results of two studies in 10 day and 21 day-old broiler birds treated subcutaneously with native LT. Subcutaneous inoculation was used since it has been reported to be as potent, if not a more potent method of lethal challenge in mice (Isaka, *et.al.* 1998). Using 16 birds and three treatment groups, no appreciable difference could be seen between untreated and treated birds. Although a few birds showed some slight diarrhea and slight hemorrhages, upon histological section no differences were observed between treated birds and untreated controls. One hallmark of a diarrhea sensitive animal is the water retention in the gut per total body weight. However, in this study the average weight of the intestines per body weight was actually lower for the controls than for the 100 ug treatment group. Based on these results there were no intense pathological responses in birds when administered LT by this method. In a second study, 84 twenty-one day-old broiler birds from the same hatch as the pilot study were treated with a broader range of native LT. Because no differences were seen between samples at the histochemical level, or gross pathology and total bird weight in the pilot study, only gross pathology and general health observations were performed in the second study. Data shown in Table 5 indicate that regardless of the treatment group, there was no pathology or overall change in the general health of the bird regardless of the LT concentration or time of observation. Although some hemorrhagic lesions were seen in some of the treated samples they were also observed for the controls, and thus were considered not to be associated with the treatment. The average weight per bird examined in the pilot study was 162 g, whereas, the average weight per bird for the challenge study was 636 g. Therefore, the LT mass to bird body weight for both studies ranged between 0.2 mg/kg to 1.2mg/kg. The reported mass to body weight ratio reported to be lethal for mice ranges between 1mg/kg body weight for CT (given IN) to 0.25mg/kg for LT (given IV). Glenn, *et al.,* 1998. *J Immunol.* **161**: 3211-3214 and Gill, 1983. For humans, as little as 0.1mg/kg is enough to cause several liters of fluid loss. The ratio tested here, by an accepted and sensitive method of challenge for LT, did not cause any indication of diarrhea compared to control

**Table 4**

| Study on Subcutaneous Inoculation of Native LT into 10-day-old Birds. | | | | | | | | | | | |
|---|---|---|---|---|---|---|---|---|---|---|---|
| Treatment | Gross Pathology | | | | | Organ weight | | | | | Bird Wt |
| | Intestine | Liver | Kidney | Spleen | Adrenal | Intestine | Liver | Kidney | Spleen | Adrenal | |
| G1 Bird 1 | N | N | N | N | nd | 18.5 | 8.1 | 1.0 | 0.2 | nd | 166.8 |
| bird 2 | N | N | N | N | nd | 19.4 | 9.95 | 1.15 | .36 | nd | 174.4 |
| bird 3 | N | N | N | N | nd | 19.1 | 11.7 | 1.95 | 0.16 | nd | 178.3 |
| bird 4 | N | N | N | N | N | 22.4 | 9.12 | 1.54 | 0.21 | nd | 204.4 |
| bird 5 | N | N | N | N | N | 16.2 | 5.6 | 1.4 | 0.16 | nd | 156.8 |
| G2 bird 6 | N | N | N | N | nd | 19.5 | 12.9 | 1.3 | 0.2 | nd | 178.4 |
| bird 7 | N | N | s.h. | N | nd | 17.8 | 12.3 | 1.3 | 0.17 | nd | 191.1 |
| bird 8 | N | s.h. | N | N | N | 15.6 | 9.2 | 1.92 | 0.18 | nd | 167.6 |
| bird 9 | D | s.h. | N | N | N | 11.4 | 8.1 | 1.51 | 0.5 | nd | 166.6 |
| bird 10 | D¹ | N | N | N | N | 14.82 | 10.8 | 2.3 | .09 | nd | 170.6 |
| G3 bird 11 | N | N | N | N | nd | 21.4 | 14.9 | 0.99 | 0.17 | nd | 186.9 |
| bird 12 | N | N | N | N | nd | 18.0 | 8.0 | 1.2 | 0.2 | nd | 167.2 |
| bird 13 | N¹ | s.h. | nd | N | N | 19.09 | 12.4 | 2.5 | 0.13 | nd | 196.1 |
| bird 14 | N¹ | N | N | N | N | 13.5 | 7.2 | 1.8 | 0.27 | nd | 142.7 |
| bird 15 | D | N | N | N | N | 13.1 | 7.4 | 1.9 | 0.11 | nd | 144.1 |
| bird 16 | D | N | N | N | N | 7.6 | 6.7 | 1.4 | 0.14 | nd | 110.3 |

| | | | | | | | | | | | |
|---|---|---|---|---|---|---|---|---|---|---|---|
| G1-group 1 non-inoculated controls; nc-no determined; N-normal; D-dehydrated; s.h.- slight hemorrhage; G2-group 2 100 ug/bird; G3-group 3 200 ug/bird; ¹Some diarrhea noticed at post mortem | | | | | | | | | | | |

**Table 5**

| Treatment of Broiler Birds Using Native LT by Subcutaneous Route. | | | | | |
|---|---|---|---|---|---|
| Treatment¹ | Bird weight (avg. gm wt) | Intestine | Liver | Kidney | Spleen |
| G1-0ug control 10h¹ | 695 | N | N | N | N |
| G1-0ug control 24h¹ | 666 | N | N | N | N |
| G1-0ug control 48 h² | 741 | m.h. 1 bird | s.he. 2 bird | N | N |
| G2- 10ug trt 10h¹ | 627 | N | N | N | N |
| G2-10ug trt 24h¹ | 662 | s.h. 1 bird | N | N | N |
| G2-10ug trt 48h³ | 756 | N | N | N | N |
| G3-50ug trt 10h¹ | 683 | N | N | N | N |
| G3-50ug trt 24h¹ | 641 | N | N | N | N |
| G3-50ug trt 48h² | 728 | N | N | N | N |
| G4-100ug trt 10h¹ | 755 | m.h. 2 birds | s.he. 2 birds | N | N |
| G4-100ug trt 24h¹ | 690 | N | N | N | N |
| G4-100ug trt 48h | 665 | m.h. 1 bird | N | m.h. 1 bird | |
| G5-200ug trt 10h¹ | 686 | N | N | N | N |
| G5-200ug trt 24h¹ | 700 | N | N | N | N |
| G5-200ug trt 48h² | 670 | N | N | N | N |
| G6-400ug trt 10hr¹ | 625 | N | s.h. | N | N |
| G6-400ug trt 24hr¹ | 534 | N | N | N | N |
| G6-400ug trt 48hr³ | 605 | N | N | N | N |

| | | | | | |
|---|---|---|---|---|---|
| ¹ 4 birds per time point per treatment group ² 6 birds per time point per treatment group ³ 5 birds per time point per treatment group m.h.- mildly hemmoragic; s.he.-small hematoma; s.h.-slightly hemorrhagic | | | | | |

### Example 15.

### Use of SLT and LT to Adjuvant Response by Intranasal/Ocular Inoculation.

This study was designed to examine the dose of LT needed to adjuvant another antigen by the intranasal/ocular route. In this study the target antigen was the hemagglutinin neuraminidase (HN) protein of the Newcastle Disease Virus described in US Patent 5,310,678. NT-1 cells were transformed in substantial accordance with Example 2 using pCHN18 as the transformation vector producing a transformed NT-1 line (CHN18) that expressed the native HN gene.

Immunogens were prepared separately by disrupting the transformed cells and lyophilizing the extracts of NT-1 cells expressing SLT, HN or null control as follows. About 1 gram of filtered cells was placed in 2 mls of buffer (DPBS and 1mM EDTA), and then sonicated for 15 to 20 seconds on ice. Sonication was performed using a Branson 450 sonifier with a replaceable microtip at output control of 8, duty cycle 60 for 20 seconds. The sonicate was then centrifuged for 16,000x for 10 minutes to remove the cell debris and the supernatant was decanted. The extract was vialed into glass serum vials, lyophilized and stored at 2-7 °C until needed. The freezed dried cell extracts were used as the inoculum for the plant derived treatments. LT holotoxin (5.92 mg/ml suspended in DPBS) derived from *E. coli* was used as a positive control.

The included vaccine inoculations were done on days 0 and 14 of the study. Extracts from CHN18 were given at 7 ug at day 0, and 18 ug at day 14. *E. coli*-derived LT was mixed with the CHN18 extract using 8 ug at day 0 and 20 ug at day 14, and the plant-derived heat labile toxin (SLT 102) was given at 0.5 ug at day 0 and 1.5 ug at day 14. Samples treated with LT or SLT were compared to treatment groups receiving a more conventional water in oil adjuvant, which were prepared by resuspending the freeze dried antigen preparation in a final concentration of 2.5% Drakeol Oil containing 0.1.65% Span 80 in DPBS with 0.5% Tween 80. Samples were mixed using two syringes and a three-way stopcock to allow suspension of the antigen in the water and oil mixture.

For intranasal/ocular inoculation, 25 ul was added per each nostril and each eye for birds younger than 10 days of age and 50 ul per nostril and eye for birds older than 10 days of age. Blood was collected on day 21, 35 and 42. At day 35 the birds were inoculated subcutaneously with inactivated native virus to simulate a challenge dose, 7 days after the simulated challenge (day 42) blood was drawn for serological analysis. The results in Table 6 show than the serological response to LT and SLT was both easily detected by 21 days into the study or 7 days after the second vaccination. The LT serological response by ELISA was much higher than that to the plant derived SLT, however, the dose used for the priming dose was over 10 fold less for the SLT than the LT. By day 35 and 42, the serological response to LT and SLT were quite high indicating that the mucosal response by intranasal and ocular route is effective when given a priming dose as low as 0.5 ug. In contrast, the response to HN protein derived from CHN18 transgenic NT1 cell lines did not give a detectable titer at day 21 by either ELISA or by HAI. However, at day 42, which was 7 days after simulated challenge with native NDV, treatments 1 and 2 both showed significant HAI serological responses that were not observed in the other treatments. The other treatments included LT and SLT (treatments 7 and 8) delivered in similar doses as treatments 1 and 2 except the were emulsified in oil and water. These results suggest that the memory response to HN could be amplified after exposure to native antigen in a simulated challenge dose. Typically a serological response to HN cannot be detected within 7 days after exposure to the antigen. Furthermore, HN antigen by itself or incombination with other mixtures did not induce a response in any of the other treatments, thus, the titers for HN shown for treatment 1 and 2 developed as a consequence of exposure of HN and LT/SLT.

**Table 6**

| Adjuvant Effect of Intranasal/Ocular Administration of LT and SLT Proteins in Birds | | | | | | | | | | |
|---|---|---|---|---|---|---|---|---|---|---|
| Treatment Group | Treatment Description | NDV ELlSA GMT | | | NUV HI GMT | | | LT ELISA GMT | | |
| | | Day 21 | Day 35 | Day 42 | Day 21 | Day 35 | Day 42 | Day 21 | Day 35 | Day 42 |
| 1 | pHN + SLT102 | 5 | Not Tested | Not Tested | 3 | 1 | 45 | 133 | 2229 | 2560 |
| 2 | pHN + E.coli LT | 1 | Not Tested | Not Tested | 2 | 1 | 28 | 4457 | 17829 | 27024 |
| 3 | pHN in Corixa (MPL/TDM Emulsion) | 2 | Not Tested | Not Tested | 1 | 1 | 3 | 4 | 17 | 67 |
| 4 | pHN in Emulsion 1 | 1 | Not Tested | Not Tested | 1 | 1 | 4 | 1 | 9 | 13 |
| 5 | pHN in Emulsion 2 | 1 | Not Tested | Not Tested | 1 | 1 | 1 | 3 | 3 | 14 |
| 6 | pHN in Emulsion 3 | 1 | Not Tested | Not Tested | 1 | 1 | 3 | 1 | 3 | 14 |
| 7 | pHN + SLT In Emulsion 1 | 1 | Not Tested | Not Tested | 2 | 1 | 1 | 352 | 2560 | 3880 |
| 8 | pHN + E. coli LT in Emulsion 1 | 5 | Not Tested | Not Tested | 5 | 1 | 4 | 844 | 8914 | 8914 |
| 9 | NT Control + SLT in Emulsion 1 | 1 | Not Tested | Not Tested | 2 | 1 | 1 | 1114 | 5881 | 3880 |
| 10 | NT Control + E. coli LT in Emulsion 1 | 1 | Not Tested | Not Tested | 1 | 1 | 2 | 3378 | 17829 | 23525 |

| | | | | | | | | | | |
|---|---|---|---|---|---|---|---|---|---|---|
| Not QA Reviewed Yet NDV ELISA Titer <50 is background NDV HI Titer <8 is background LT ELISA Titer <10 is background | | | | | | | | | | |

The results in Table 7 were generated using subcutaneous (SQ) inoculations of LT in combination with HN. Vaccine preparation was as described for Table 6. Vaccine was administered by inoculating in the thigh web in this study

Using two inoculations at day 0 and 14 of the study. A simulated challenge dose with inactivated NDV was administered at day 35. The results shown in Table 7 demonstrate that serological response to LT also results from administration of SQ inoculation, and that enhancement of HN serological response was observed when co-administered.

**Table 7**

| Subcutaneous Inoculation of Chickens with LT and Plant-derived HN | | | | | | | |
|---|---|---|---|---|---|---|---|
| Treatment Group | Treatment Group Description | NDV HI GMT | | | LT ELISA GMT | | |
| | | Day 21 | Day 35 | Day 42 | Day 21 | Day 35 | Day 42 |
| 7 | pHN (20 ug) + SLT (2.5 ug) m Emulsion 1 | 24 | 7 | 38 | 24 | 17 | 16 |
| 8 | pHN (20 ug) + E.coli LT (20 ug) in Emulsion 1 | 239 | 120 | 128 | 557 | 735 | 1114 |
| 9 | NT control + SLT (2.5 ug) in Emulsion 1 | 1 | 1 | 19 | 27 | 190 | 190 |
| 10 | NT control + E.coli LT (20 ug) m Emusion 1 | 1 | 2 | 20 | 1613 | 2032 | 3225 |

LT also stimulated a response by *in ovo* inoculation. In table 8, LT derived from *E. coli* was administered by inoculation through the air sac and into the amnion cavity. Fertile eggs were candled at day 18 of incubation and only healthy embryos were indentified for inoculation. The injection site was swabbed with alcohol directly over the air cell of the egg. A hole was gently punched using an egg punch and a 22 gauge needle 1 ½ inches long was inserted through the hole. Up to 0.3 ml of inoculum was deposited into the amnion cavity just beneath the air sac membrane. The eggs were then transferred to the hatchery from day 18-21. Fourteen days after hatch the birds were boosted with LT and blood was analyzed by serological response at day 21. Serological response was not observed with a single inoculation within 7 days unless a priming dose had previously been administered. The results indicate that LT inoculated *in ovo* effectively primes an 18-day old embryo.

**Table 8**

| *In Ovo* immunization | | |
|---|---|---|
| Treatment Group Description | Day 21 Serology GMT | |
| | # hatched | LT ELISA |
| NT control in Emulsion 2 | 5 | 1 |
| NT control + E.coli LT (10 ug) | 3 | 1016 |

The ordinarily skilled artisan will readily recognize or ascertain many equivalents to specific embodiments of the invention described and illustrated herein based on this specification, the prior art or mere routine experimentation.

## Claims

1. A composition comprising the adjuvant native *E. coli* heat-labile toxin (LT) for use in vaccinating a broiler chicken wherein the composition is adapted for administration via the oral or subcutaneous route.

2. The composition of Claim 1 further comprising an immunoprotective antigen effective in a broiler chicken.

3. The composition of Claim 2 wherein the immunoprotective antigen is derived from the group consisting of a viral, bacterial, or fungal pathogen.

4. The composition of Claim 3 wherein the immunoprotective antigen is selected from the group consisting of known immunoprotective antigens of Newcastle disease, avain influenza, infectious bursal disease, coccidiosis, necrotic enteritis, airsacculitis, cellulitis, chicken anemia, larygnorhinotracheitis, infectious bronchitis, and Marek's disease.

5. The composition of Claim 4 wherein the immunoprotective antigen is selected from the group consisting of Newcastle disease virus hemaglutinin neuraminidase protein and avian influenza hemagglutinin protein.

6. The composition of Claim 1 wherein the adjuvant is produced by an *E. coli* bacterium.

7. The composition of Claim 1 wherein the adjuvant is produced by a transgenic plant.

8. The composition of Claim 2 wherein the immunoprotective antigen is produced in a transgenic plant.

9. The composition of Claim 2 wherein the adjuvant and the immunoprotective antigen are produced in a transgenic plant.

10. The composition of Claim 2 wherein the adjuvant and the immunoprotective antigen are produced in a single transgenic plant.

11. The use of a composition according to any of Claims 1 to 5 for the manufacture of a medicament for vaccinating a broiler chicken against an avian disease.

12. A method for preparing a vaccine for protecting a broiler chicken against an avian disease which comprises mixing an effective amount of native LT with an effective amount of an immunoprotective antigen known to elicit an immune response in a bird, wherein the vaccine is adapted for administration via the oral or subcutaneous route.

13. The method of Claim 12 wherein the immunoprotective antigen is selected from the group consisting of known immunoprotective antigens of Newcastle disease, avain influenza, infectious bursal disease, coccidiosis, necrotic enteritis, airsacculitis, cellulitis, chicken anemia, larygnorhinotracheitis, infectious bronchitis, and Marek's disease.

14. The method of Claim 12 wherein the immunoprotective antigen is selected from the group consisting of Newcastle disease virus hemaglutinin neuraminidase protein and avian influenza hemagglutinin protein.

15. The method of Claim 12 wherein the immunoprotective antigen is the Newcastle disease virus hemaglutinin neuraminidase protein.

## Patentansprüche

1. Zusammensetzung, die das Adjuvans natives hitzelabiles *E. coli*-Toxin (LT) umfasst, zur Verwendung für die Impfung von Hähnchen, wobei die Zusammensetzung für die Verabreichung auf oralem oder subkutanem Weg geeignet Ist.

2. Zusammensetzung nach Anspruch 1, die weiterhin ein Immunprotektives Antigen umfasst, das in Hähnchen wirksam ist.

3. Zusammensetzung nach Anspruch 2, wobei das immunprotektive Antigen aus der Gruppe bestehend aus einem viralen, bakteriellen oder Pilzpathogen stammt.

4. Zusammensetzung nach Anspruch 3, wobei das Immunprotektive Antigen aus der Gruppe bestehend aus den bekannten immunprotektiven Antigenen der Newcastle-Krankheit, Vogelgrippe, infektiösen Bursitis der Hühner, Kokzidiose, nekrotischen Enteritis, Entzündung der Schlelinhaut der Lungenalveolen von Vögeln (Airsacculitis), Cellulitis, Hühner-Anärnie, Laryngo-Rhlno-Tracheltis, Infektiösen Bronchitis und der Marek-Krankheit ausgewählt ist.

5. Zusammensetzung nach Anspruch 4, wobei das immunprotektive Antigen aus der Gruppe bestehend aus dem Hämagglutinin-Neuraminidase-Proteln des Newcastle-Disease-Virus und dem Hämagglutinin-Proteln der Vogelgrippe ausgewählt ist.

6. Zusammensetzung nach Anspruch 1, wobei das Adjuvans von einem *E. coli-*Bakterium produziert wird.

7. Zusammensetzung nach Anspruch 1, wobei das Adjuvans von einer transgenen Pflanzo produziert wird.

8. Zusammensetzung nach Anspruch 2, wobei das immunprotektive Antigen In einer transgenen Pflanze produziert wird.

9. Zusammensetzung nach Anspruch 2, wobei das Adjuvans und das immunprotektive Antigen in einer transgenen Pflanze produziert werden.

10. Zusammensetzung nach Anspruch 2, wobei das Adjuvans und das immunprotektive Antigen in einer einzelnen transgenen Pflanze produziert werden.

11. Verwendung einer Zusammensetzung nach einem der Ansprüche 1 bis 6 zur Herstellung eines Medikaments, um Hähnchen gegen eine Vogelkrankheit zu impfen.

12. Verfahren zur Herstellung eines Impfstoffs, um Hähnchen gegen eine Vogelkrankheit zu schützen, wobei das Verfahren es umfasst, eine wirksame Menge an nativem LT mit einer wirksamen Menge eines immunprotektiven Antigens, von dem bekannt Ist, dass es in einem Vogel eine Immunantwort hervorruft, zu mischen, wobei der Impfstoff für die Verabreichung auf oralem oder subkutanem Weg geeignet ist.

13. Verfahren nach Anspruch 12, wobei das irnmunprotoktive Antigen aus der Gruppe bestehend aus den bekannten immunprotektlven Antigenen der Newcastle-Krankheit, Vogelgrippe, Infektiösen Bursitis der Hühner, Kokzidiose, nekrotischen Enteritis, Entzündung der Schleimhaut der Lungenalveolen von Vögeln (Airsacculitis), Cellulitis, Hühner-Anämle, Laryngo-Rhino-Tracheitis, lnfektiösen Bronchitis und der Marek-Krankheit ausgewählt ist.

14. Verfahren nach Anspruch 12, wobei das immunprotektive Antigen aus der Gruppe bestehend aus dem Hämagglutinin-Neuraminidase-Protcin des Newcastle-Disease-Virus und Wämagglutinin-Protein der Vogelgrippe ausgewählt ist.

15. Verfahren nach Anspruch 12, wobei das immunprotektive Antigen das Hämagglutinin-Neuraminidase-Protein des Newcastle-Disease-Virus ist.

## Revendications

1. Composition comprenant, en tant qu'adjuvant, de la toxine thermolabile (TTL) native d'*E. coli* et destinée à être employée dans la vaccination de poulets de chair, laquelle composition est adaptée pour pouvoir être administrée par voie orale ou par voie sous-cutanée.

2. Composition conforme à la revendication 1, comprenant en outre un antigène immunoprotecteur efficace chez des poulets de chair.

3. Composition conforme à la revendication 2, dans laquelle l'antigène imnunoprotecteur dérive d'un agent pathogène viral, bactérien ou fongique.

4. Composition conforme à la revendication 3, pour laquelle l'antigène immunoprotecteur a a été choisi dans l'ensemble formé par les antigènes immunoprotecteurs connus contre les maladies suivantes : maladie de Newcastle, grippe aviaire, bursite infectieuse, coccidiose, entérite nécrosante, mycoplasmose, cellulite, anémie du poulet, laryngo-rhino-trachéite, bronchite infectieuse, et maladie de Marek.

5. Composition conforme à la revendication 4, pour laquelle l'antigène immunoprotecteur a été choisi dans l'ensemble formé par la protéine hémagglutinine-neuraminidase du virus de la maladive de Newcastle et la protéine hemagglutinine du virus de la grippe aviaire.

6. Composition conforme à la revendication 1, dont l'adjuvant a été produit par une bactérie *E. coli.*

7. Composition conforme à la revendication 1, dont l'adjuvant a été produit chez une plante transgénique.

8. Composition conforme à la revendication 2, dont l'antigène immunoprotecteur a été produit une plante transgénique.

9. Composition conforme à la revendication 2, dont l'adjuvant et l'antigène immunoprotecteur ont été produits chez une plante transgénique.

10. Composition conforme à la revendication 2, dont l'adjuvant et l'antigène immunoprotecteur ont été produits chez une seule plante transgénique.

11. Utilisation d'une composition conforme à l'une des revendications 1 à 5 en vue de la fabrication d'un médicament conçu pour la vaccination de poulets de chair contre une maladie aviaire.

12. Procède de préparation d'un vaccin conçu pour protéger des poulets de chair contre une maladie aviaire, lequel procédé comporte le fait de mélanger une quantité efficace de toxine thermolabile native avec une quantité efficace d'un antigène immunoprotecteur dont on sait qu'il provoque une réponse immune chez un oiseau, ledit vaccin étant adapte pour pouvoir être administré par voie orale ou par voie sous-cutanée.

13. Procédé conforme à la revendication 12, pour lequel l'antigène immunoprotecteur a été choisi dans l'ensemble constitué par les antigènes immunoprotecteurs connus contre les maladies suivantes : maladie de Newcastle, grippe aviaire, bursite infectieuse, coccidiose, entérite nécrosante, mycoplasmose, cellulite, anémie du poulet, laryngo-rhino-trachéite, bronchite infectieuse, et maladie de Marek.

14. Procède conforme à la revendication 12, pour lequel l'antigène immunoprotecteur a été choisi dans l'ensemble constitué par la protéine hémagglutinine-neuraminidase du virus de la maladie de Newcastle et la protéine hémagglutinine du virus de la grippe aviaire.

15. Procédé conforme à la revendication 12, dans lequel l'antigéne immunoprotecteur est la protéine hémagglutinine-neuraminidase du virus de la maladie de Newcastle,
